# EUROPEAN PATENT APPLICATION

(11) **EP 4 458 942 A1**
(43) Date of publication of application: **06.11.2024**
(21) Application number: 22916675.6
(22) Date of filing: 27.12.2022
(51) Int. Cl.: C12M 1/00, C12M 1/34

(54) **CULTURING APPARATUS AND CULTURING SYSTEM INCLUDING SAME**

(30) Priority: 29.12.2021 KR 20210191575
(71) Applicant: CJ CheilJedang Corporation, Seoul 04560 (KR)
(72) Inventor: SEO, Dong Woo, Seoul 04560 (KR); KIM, Yeung Tak, Seoul 04560 (KR)
(74) Representative: Bandpay & Greuter
(86) International application number: PCT/KR2022/021338
(87) International publication number: WO 2023/128534

(57) **Abstract**

A culturing system according to one embodiment of the present invention may comprise: a culturing chamber having a culturing space, which is an inner space formed to accommodate a culture chamber, so that the culture plate enters and exits; a seed supply unit configured to supply a seed to the culture plate; a fluid supply unit formed to supply water, moisture, and air to adjust the temperature and humidity of the culture space; and a processor electrically connected to the fluid supply unit, wherein the processor controls the fluid supply unit such that at least one of the temperature and humidity of the culturing space is adjusted.

## Description

### [Technical Field]

The present invention relates to a culturing apparatus and a culturing system including the culturing apparatus.

### [Background Art]

In order to culture seeds such as cells and strains, it is necessary to maintain an environment suitable for culturing, and the culturing environment may be determined by various factors such as temperature, humidity, pressure, and culturing time.

Researches are continuously being conducted on a culturing apparatus and a culturing system that may control and maintain the culturing environment. In particular, to satisfy optimal culture conditions that vary depending on types or characteristics of seeds, a culturing apparatus and a culturing system capable of controlling factors affecting a culturing environment are being actively developed.

According to the related art, since components required for seed supply are replaced and used, there are limits to real-time control of factors such as temperature, humidity, and pressure, and it takes a long time to culture. In addition, according to the related art, spread does not occur smoothly when culturing aggregated cells, strains, seeds, etc., making it difficult to secure uniform quality.

### [Disclosure]

### [Technical Problem]

The problem to be solved by the present invention is to provide a culturing apparatus and a culturing system capable of supplying seeds in an effective manner and smoothly spreading cells, strains, seeds, etc., within the culturing apparatus. In addition, the present invention provides a culturing apparatus and a culturing system capable of effectively controlling environmental factors necessary for culturing, such as temperature, humidity, and pressure.

### [Technical Solution]

According to an embodiment of the present invention, a culturing apparatus may include a culturing chamber having a culturing space which is an internal space in which a culturing plate is accommodated, a culturing door coupled to the culturing chamber to open and close an opening formed in the culturing chamber to allow the culturing plate to enter and exit, a supply line unit formed in at least one of the culturing chamber and the culturing door to control a temperature, moisture supply, and seed supply of the culturing space, respectively, and a fan part formed in at least one of the culturing chamber and the culturing door to spread seed to the culturing plate.

According to another embodiment of the present invention, a culturing system includes a culturing chamber having a culturing space which is an internal space in which a culturing plate is accommodated, a culturing door coupled to the culturing chamber to open and close an opening formed in the culturing chamber to allow the culturing plate to enter and exit, a seed supply unit configured to supply seed to the culturing plate, a fluid supply unit configured to supply water, moisture, and air to control a temperature and humidity of the culturing space, and a processor electrically connected to the fluid supply unit, wherein the processor may control the fluid supply unit to control at least one of the temperature and humidity of the culturing space.

### [Advantageous Effects]

According to the embodiments of the present invention, it is possible to efficiently supply seeds by effectively supplying and spreading the seeds within the culturing apparatus.

According to the embodiments of the present invention, it is possible to stably and effectively control the environmental factors necessary for culturing, such as temperature, humidity, and pressure.

### [Brief Description of the Drawings]

FIG. 1 is a diagram illustrating a culturing apparatus according to an embodiment of the present invention.
FIG. 2 is a diagram illustrating the culturing apparatus according to the embodiment of the present invention from top.
FIG. 3 is a diagram illustrating an inside of a culturing chamber according to an embodiment of the present invention.
FIG. 4 is a diagram illustrating in detail the inside of the culturing chamber according to the embodiment of the present invention.
FIG. 5 is a diagram illustrating the culturing apparatus according to the embodiment of the present invention from the side.
FIG. 6 is a diagram illustrating an internal flow of the culturing apparatus according to the embodiment of the present invention.
FIG. 7 is a diagram illustrating a fan part of the culturing apparatus according to the embodiment of the present invention.
FIG. 8 is a diagram illustrating a flow of water supplied to control a temperature of the culturing apparatus according to the embodiment of the present invention.
FIG. 9 is a diagram illustrating a culturing system including a culturing apparatus according to an embodiment of the present invention.
FIG. 10 is a diagram illustrating a seed supply unit according to an embodiment of the present invention.

### [Mode for Invention]

This application claims the benefit of priority based on Korean Patent Application No. 10-2021-0191575, dated December 29, 2021, and all contents disclosed in the document of the Korean Patent Application are included as part of this specification.

Hereinafter, preferred embodiments of the present invention will be described in detail with reference to the accompanying drawings so that those skilled in the art to which the present invention pertains may easily practice the present invention. However, the present invention may be implemented in several different forms and is not limited to embodiments below.

In order to clearly describe the present invention, detailed descriptions of parts unrelated to the description or related known technologies that may unnecessarily obscure the gist of the present invention have been omitted. In this specification, in adding reference numerals to components in each drawing, identical or similar reference numerals are assigned to identical or similar components throughout the specification.

In addition, the terms and words used in the present specification and the claims are not to be construed as being limited to general or dictionary meanings, and are to be construed as meaning and concepts meeting the technical idea of the present invention based on a principle that the present inventors may appropriately define the concepts of the terms in order to describe their own inventions in the best manner.

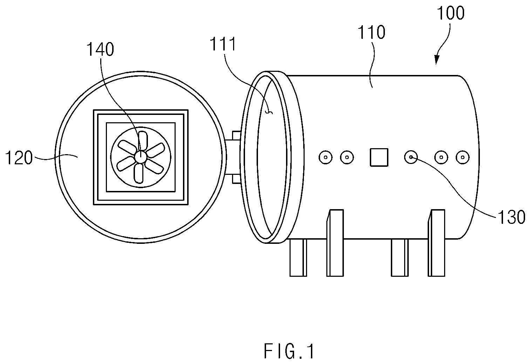

FIG. 1 is a diagram illustrating a culturing apparatus 100 according to an embodiment of the present invention.

The culturing apparatus 100 may include a culturing chamber 110. For example, the culturing chamber 110 may form an outside of a culturing space 111 where seeds may be cultured.

The culturing apparatus 100 may include the culturing space 111. For example, the culturing space 111 may be an internal space in which the culturing plate 200 may be accommodated inside the culturing chamber 110.

The culturing apparatus 100 may include a culturing door 120. For example, the culturing door 120 may form the outside of the culturing space 111. The culturing door 120 may be coupled to the culturing chamber 110 to open and close an opening formed in the culturing chamber 110 to allow the culturing plate 200 to enter and exit.

The culturing chamber 110 and the culturing door 120 may close the culturing space 111 from the outside. For example, a packing member may be arranged between the culturing chamber 110 and the culturing door 120, and a volume of the packing member may increase to close the culturing space 111 from the outside.

The culturing chamber 110 and the culturing door 120 may open the culturing space 111. For example, the packing member may be arranged between the culturing chamber 110 and the culturing door 120, and a volume of the packing member may decrease to open the culturing space 111.

The culturing chamber 110 may include a fan part 140. For example, the fan part 140 may generate flow in the culturing space 111. In addition, the fan part 140 may scatter (or spread) seeds supplied to the culturing space 111. The fan part 140 may include a plurality of fans.

The culturing apparatus 100 may include a supply line unit 130 that is provided to supply water, moisture, air, and seeds to the culturing space 111. The supply line unit 130 may include a plurality of supply lines.

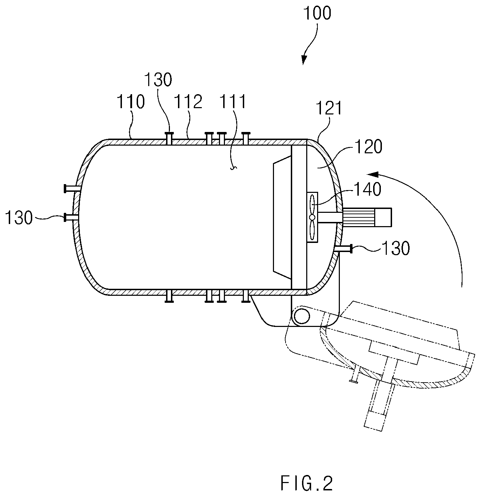

FIG. 2 illustrates the culturing apparatus 100 according to the embodiment of the present invention from top.

The culturing apparatus 100 may include the culturing chamber 110 and the culturing door 120.

The culturing chamber 110 may include the supply line unit 130. For example, the supply line unit 130 may be configured to control temperature, moisture supply, and seed supply, respectively. As a specific example, the supply line unit 130 may be formed of a plurality of supply lines, and may perform supply water for temperature control, moisture supply for humidity control, and seed supply for culturing. Some of the plurality of supply lines included in the supply line unit 130 may include spray nozzles.

The culturing chamber 110 may include a chamber circulation channel 112. For example, the culturing chamber 110 may include a chamber circulation channel 112 configured to circulate water supplied for temperature control outside the culturing space 111 covered by the culturing chamber 110. The outside where the water circulates may be in the form of a shell surrounding the culturing space 111.

The culturing door 120 may be rotatably coupled to the culturing chamber 110. For example, the culturing door 120 may be coupled to a portion of the culturing chamber 110 and rotate based on the opening to open the culturing space 111.

The culturing door 120 is rotatably coupled to the culturing chamber 110 to open and close the culturing space 111.

The fan part 140 may be coupled to the culturing door 120. For example, the fan part 140 may be coupled to one side portion of the culturing door 120. The fan part 140 may be configured to be located within the culturing space 111 when the culturing door 120 closes the culturing space 111.

The fan motor 141 may be coupled to the culturing door 120. For example, the fan motor 141 may be coupled to the other side portion of the culturing door 120 and arranged on an opposite side to the fan part 140 based on a center of the culturing door 120. The fan motor 141 may be electrically or operationally connected to the fan part 140 to provide a driving force for the rotation of the fan part 140.

The culturing door 120 may include a water supply line 133 in the supply line unit 130. For example, the culturing door 120 may include the water supply line 133 provided to supply water for temperature control.

The culturing door 120 may include a door circulation channel 121. For example, the culturing door 120 may include the door circulation channel 121 configured to circulate water supplied for temperature control outside the culturing space 111 covered by the culturing chamber 120. The outside where the water circulates may be in the form of the shell surrounding the culturing space 111.

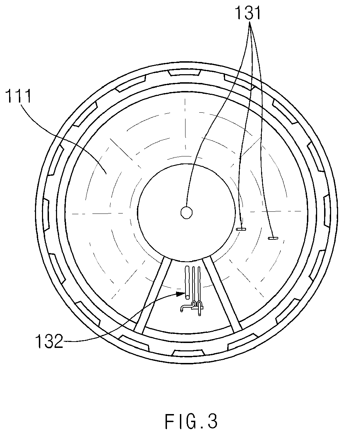

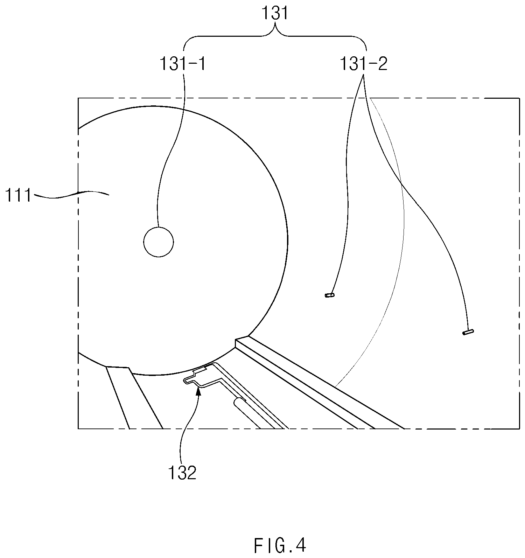

FIGS. 3 and 4 illustrate the inside of the culturing chamber 110 according to the embodiment of the present invention.

The culturing chamber 110 may include the supply line unit 130. For example, the supply line unit 130 may be formed of a plurality of supply lines. The supply line unit 130 may include a seed supply line 131 provided to supply seeds to be cultured in the culturing space 111, and a moisture supply line 132 provided to supply moisture and air necessary for culturing. The supply line unit 130 may further include a water supply line 133 provided to supply water to the culturing apparatus 100 or the culturing door 120 to control the temperature of the culturing space 111. However, it may not be limited thereto.

The seed supply line 131 may include a plurality of seed supply lines. For example, the seed supply line 131 may include a first seed supply line 131-1 provided on an inner rear surface located on the opposite side of the opening based on the center of the culturing chamber 110 and a second seed supply line 131-2 provided on an inner side surface of the culturing chamber 110. The positions of the first seed supply line 131-1 and the second seed supply line 131-2 described above are examples and may not be limited thereto.

The first seed supply line 131-1 may be provided to supply at least solid seeds, which are seeds in a solid state. For example, the first seed supply line 131-1 may be provided to supply the solid seeds. However, it may not be limited thereto.

The second seed supply line 131-2 may supply the solid seeds from the inner side surface of the culturing chamber 110. The second seed supply lines 131-2 may be provided in plurality on the inner side surface of the culturing chamber 110. However, it may not be limited thereto.

The first seed supply lines 131-1 and second seed supply lines 131-2 may be formed in plurality to have different heights. For example, the first seed supply line 131-1 may be formed of a plurality of supply lines having different heights in order to easily spread seeds to the culturing plate 200 accommodated in the culturing space 111. In addition, the second seed supply line 131-2 may also be formed of a plurality of supply lines having different heights in order to easily spread seeds to the culturing plate 200 accommodated in the culturing space 111.

The seed supply line 131 may include a spray nozzle, and the seed may be easily spread by the spray nozzle. When the seed supply line 131 includes a plurality of seed supply lines, a plurality of injection nozzles may also be provided. The seed supply line 131 may include a non-powered fan, and when the seeds are supplied to the culturing space 111, the seeds may be easily spread by a rotation of the non-powered fan.

A moisture supply line 132 may be formed on an inner lower surface of the culturing chamber 110 to supply moisture and air for humidity control. For example, the moisture supply line 132 may be provided to supply moisture and air from the inner lower surface of the culturing chamber 110 to the culturing space 111 to control the humidity of the culturing space 111. The moisture supply line 132 may include a spray nozzle, and the spray nozzle may facilitate the spread of moisture.

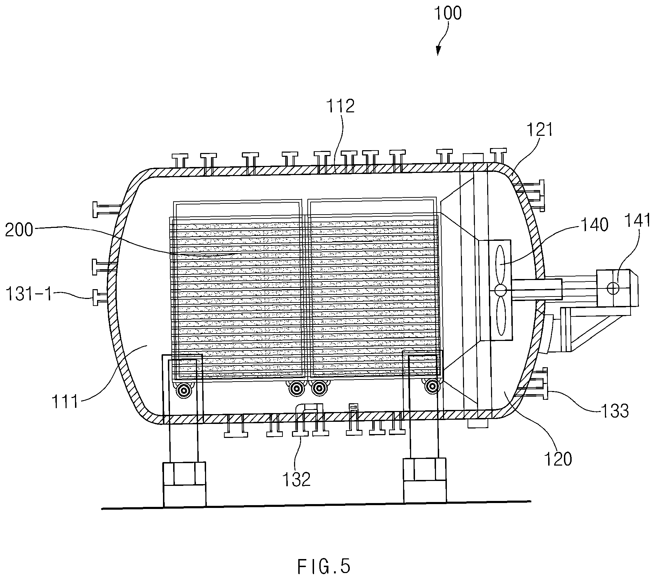

FIG. 5 is a diagram illustrating the culturing apparatus according to the embodiment of the present invention from the side.

The culturing apparatus 100 may include the culturing chamber 110 and the culturing door 120.

A culturing plate 200 may be accommodated in the culturing space 111 that is the internal space of the culturing chamber 110. For example, the culturing plate 200 may be accommodated in the culturing space 111 through the opening of the culturing chamber 110. The culturing plate 200 may have a form in which a plate is formed in multi-stage in order to secure the maximum capacity of seeds to be cultured. The culturing plate 200 may include wheels provided at the lower portion to facilitate movement. Guidelines that may guide the wheels may be formed on the inner lower surface of the culturing chamber 110.

The culturing chamber 110 may include the supply line unit 130. The supply line unit 130 may be configured to control temperature, moisture supply, and seed supply, respectively. For example, the supply line unit 130 may be formed of a plurality of supply lines, and the plurality of supply lines may perform water supply for temperature control, moisture supply for humidity control, and seed supply for culturing to the culturing space 111.

The supply line unit 130 may include the seed supply line 131 provided to supply seeds. The seed supply line 131 may include the first seed supply line 131-1 and the second seed supply line 131-2. The first seed supply line 131-1 may be formed on the inner rear surface of the culturing apparatus 100, and the second seed supply line 131-2 may be formed on the inner side surface of the culturing apparatus 100. However, it may not be limited thereto.

The supply line unit 130 may include the moisture supply line 132 provided to supply moisture and air. For example, the moisture supply line 132 may be formed on the inner lower surface of the culturing chamber 110 and may supply moisture and air to the culturing space 111.

The culturing chamber 110 may include a chamber circulation channel 112. For example, the culturing chamber 110 may include the chamber circulation channel 112 configured to circulate water supplied for temperature control outside the culturing space 111. The chamber circulation channel 112 may be formed on a shell-shaped outside of the culturing chamber 110 in the form surrounding the culturing space 111.

The culturing door 120 is rotatably coupled to the culturing chamber 110 to open and close the culturing space 111. For example, the culturing door 120 may be rotatably coupled to the culturing chamber 110. The culturing door 120 is rotatably coupled to a portion of the culturing chamber 110 and rotates based on the opening, thereby opening or closing the culturing space 111.

The culturing apparatus 100 may include the fan part 140. For example, the fan part 140 may be coupled to the culturing door 120. The fan part 140 may be coupled to one side portion of the culturing door 120. The fan part 140 may be configured to be located within the culturing space 111 when the culturing door 120 closes the culturing space 111.

The culturing apparatus 100 may include the fan motor 141. For example, the fan motor 141 may be coupled to the culturing door 120. The fan motor 141 may be coupled to the other side portion of the culturing door 120 and arranged on an opposite side to the fan part 140 based on a center of the culturing door 120. The fan motor 141 may be electrically or operationally connected to the fan part 140 to provide a driving force for the rotation of the fan part 140.

The culturing door 120 may include a water supply line 133 in the supply line unit 130. For example, the culturing door 120 may include the water supply line 133 provided to supply water for temperature control. However, it may not be limited thereto.

The culturing door 120 may include the door circulation channel 121. For example, the culturing door 120 may include the chamber circulation channel 121 configured to circulate water supplied for temperature control outside the culturing space 111. The door circulation channel 121 may be formed on the outside of the culturing door 120 in the form of the shell surrounding the opening so that water circulates around the outside of the opening side of the culturing space 111.

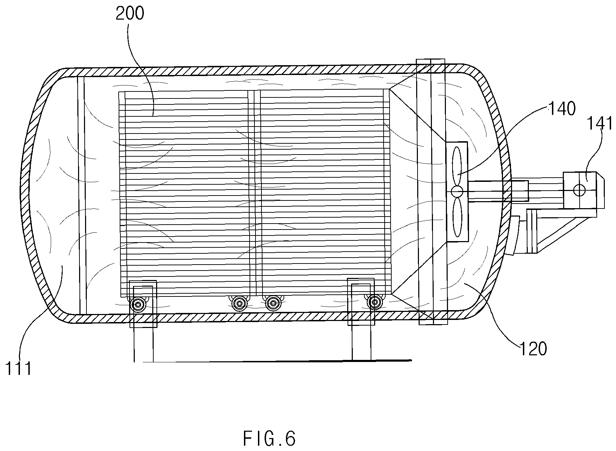

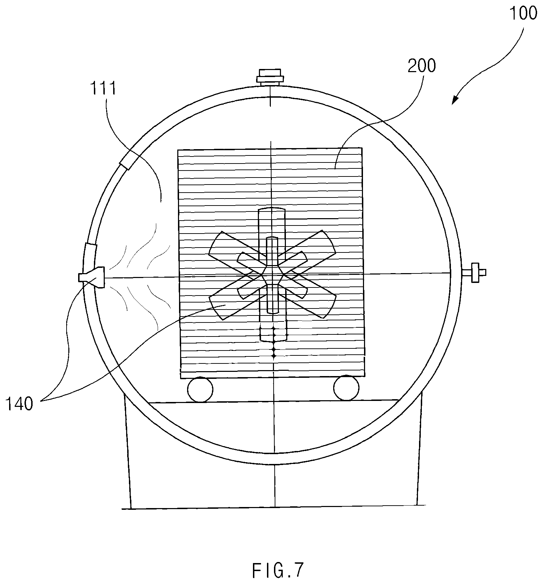

FIG. 6 illustrates the internal flow of the culturing apparatus according to the embodiment of the present invention, and FIG. 7 illustrates the fan part of the culturing apparatus according to the embodiment of the present invention.

The culturing apparatus 100 may include the fan part 140. In addition, the fan part 140 may include a plurality of fans. For example, the fan part 140 may include a first fan provided in the culturing door 120 to generate a flow and a second fan provided in the culturing chamber 110 to generate the flow.

Referring to FIG. 6, the first fan in the fan part 140 may be provided in the culturing door 120. For example, the culturing door 120 may include the first fan, and as the first fan rotates, the flow of air may occur in the culturing space 111, and the environmental conditions, such as temperature, pressure, and humidity within the culturing space 111 may be maintained uniformly.

Referring to FIG. 7, the second fan in the fan part 140 may be provided in the culturing chamber 110. For example, a second fan may be provided on the inner side surface of the culturing chamber 110. The second fan may be a non-powered fan, and a flow may be formed in the culturing space 111 by rotating the second fan while supplying seeds from the outside according to negative pressure. The second fan may be connected to the seed supply line 131, and the second fan may rotate as seeds are supplied to the culturing space 111 through the seed supply line 131, thereby uniformly scattering (or spreading) the seeds to the culturing plate 200 in the culturing space 111.

The fan part 140 may be provided to control a rotation speed. For example, the rotation speed of the fan part 140 may be controlled to control the degree of flow or scattering (or spreading) . When the fan part 140 includes the first fan provided in the culturing door 120 to generate a flow and the second fan provided in the culturing chamber 110 to generate a flow, the fan part 140 may be provided to control the rotation speed of each of first fan and second fan.

The culturing apparatus 100 includes the fan part 140, thereby generating the flow of air and uniform diffusion of seeds in the culturing space 111, and controlling or maintaining the environmental conditions such as temperature, pressure, and humidity within the culturing space 111.

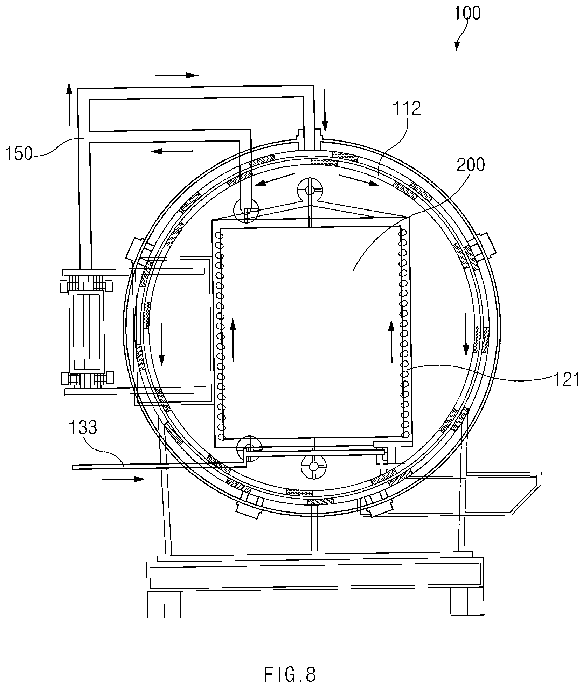

FIG. 8 is a diagram illustrating the flow of water supplied to control the temperature of the culturing apparatus according to the embodiment of the present invention.

The culturing apparatus 100 may be supplied with water for temperature control. For example, the culturing apparatus 100 may be supplied with water (e.g., hot water or cold water) to control the temperature of the culturing space 111. When hot water is supplied to the culturing apparatus 100, the temperature of the culturing space 111 may increase, and when cold water is supplied to the culturing apparatus 100, the temperature of the culturing space 111 may be lowered.

The culturing apparatus 100 may receive water through the water supply line 133. The water supply line 133 may be formed in the culturing door 120. The water supplied to the culturing door 120 through the water supply line 133 may be circulated through the door circulation channel 121. The door circulation channel 121 may be configured to surround a portion of the culturing space 111 in the culturing door 120. The water circulated through the door circulation channel 121 may be transmitted to the chamber circulation channel 112 through the connection channel 150.

The connection channel 150 may be configured to connect the door circulation channel 121 and the chamber circulation channel 112. For example, the water circulated in the door circulation channel 121 may be transmitted to the connection channel 150 connected to the door circulation channel 121 and circulated in the connection channel 150 transmitted to the connection channel 150. The water circulated in the connection channel 150 may be transmitted to the chamber circulation channel 112.

The water transmitted to the chamber circulation channel 112 may be circulated in the culturing chamber 110. For example, the water transmitted to the chamber circulation channel 112 may be circulated along the chamber circulation channel 112 formed in the culturing chamber 110 in the form surrounding the culturing space 111.

The water (e.g., hot or cold water) transmitted to the culturing apparatus 100 through the water supply line 133 may be circulated in the culturing door 120 and the culturing chamber 110, which form the outside of the culturing space 111, thereby adjusting the temperature of the culturing space 111.

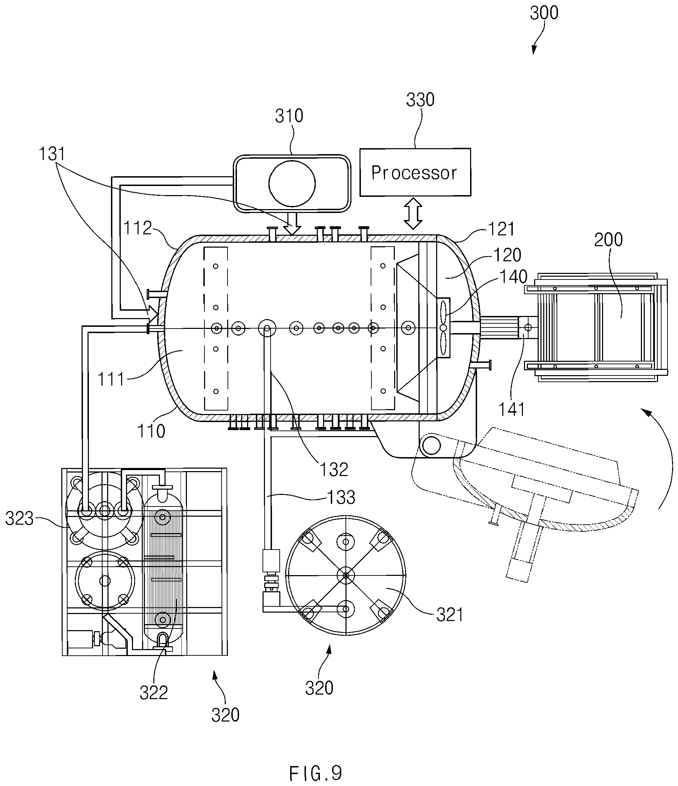

FIG. 9 illustrates a culturing system 300 including the culturing apparatus 100 according to the embodiment of the present invention.

The culturing system 300 may include the culturing apparatus 100. The description of the culturing apparatus 100 described above with reference to FIGS. 1 to 8 may be equally applied to the culturing apparatus 100 included in the culturing system 300.

The culturing system 300 may include a seed supply unit 310.

The seed supply unit 310 may be provided to supply seeds for culturing to the culturing apparatus 100. For example, the seed supply unit 310 may supply seeds to be cultured to the seed supply line 131. The seed supply unit 310 may supply seeds to the culturing plate 200 that may be accommodated in the culturing space 111 through the seed supply line 131. The seed supply unit 310 may include a plurality of seed supply units according to the type or characteristics of seeds. The seed supply unit 310 may include one of a solid seed supply unit that supplies solid seeds, which are seeds in a solid state, and a liquid seed supply unit that supplies liquid seeds, which are seeds in a liquid state. The liquid seeds may include not only seeds formed only from liquid, but also seeds formed from a mixture of liquid and solid.

The culturing system 300 may include a fluid supply unit 320.

The fluid supply unit 320 may be configured to supply water, moisture, and air to control the temperature and humidity of the culturing space 111.

The fluid supply unit 320 may include a water supply unit 321 provided to supply water for temperature control of the culturing space 111. For example, the water supply unit 321 may control the temperature of the water supplied. When it is intended to increase the temperature of the culturing space 111, the water supply unit 321 may supply hot water (or high temperature water) to the culturing apparatus 100 through the water supply line 133. When it is intended to lower the temperature of the culturing space 111, the water supply unit 321 may supply cold water (or low temperature water) to the culturing apparatus 100 through the water supply line 133.

Although not illustrated, the fluid supply unit 320 may supply moisture for humidity control to the culturing space 111 through the moisture supply line 132 using a separate moisture supply unit.

Although not illustrated, the fluid supply unit 320 may also supply air to the culturing space 111 using a separate air supply unit.

The fluid supply unit 320 may include a condenser 322 and a pressure pump 323. For example, the condenser 322 and the pressure pump 323 may be connected to the culturing space through the connection line, and may control the internal pressure of the culturing space 111. The condenser 322 and the pressure pump 323 may form the negative pressure by lowering the internal pressure of the culturing space 111. The condenser 322 and the pressure pump 323 may lower the internal pressure of the culturing space 111, thereby controlling the internal pressure of the culturing space 111 to correspond to vacuum.

When the negative pressure is formed in the culturing space 111, the culture seeds supplied by the seed supply unit 310 may move to the culturing space 111 through the seed supply line 131 due to the pressure difference. Seeds may be supplied to the culturing plate 200 accommodated in the culturing space 111.

The culturing system 300 may include a processor 330.

The processor 330 may be electrically connected to at least one of the seed supply unit 310 and the fluid supply unit 320. The processor 330 may control operations of components included in at least one of the seed supply unit 310 and the fluid supply unit 320.

The processor 330 is electrically connected to the culturing apparatus 100 and may control the components of the culturing apparatus 100. For example, the processor 330 may control the rotation speed of the fan part 140 included in the culturing apparatus 100. However, it may not be limited thereto.

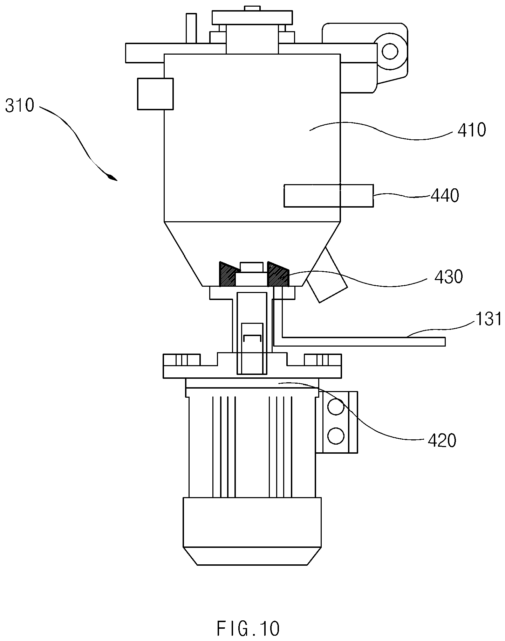

FIG. 10 illustrates the seed supply unit 310 according to the embodiment of the present invention. The seed supply unit 310 according to FIG. 10 has been described on the premise that it is the solid seed supply unit, but may not be limited thereto.

The seed supply unit 310 may include a solid seed storage unit 410. The solid seed storage unit 410 may be provided to store the solid seeds and may be connected to the seed supply line 131. When the negative pressure is formed in the culturing space 111, the solid seeds stored in the solid seed storage unit 410 may move to the culturing space 111 through the seed supply line 131 due to the pressure difference.

The seed supply unit 310 may include a mixer 420, and the mixer 420 may include a blade 430. For example, the solid seeds stored in the solid seed storage unit 410 may be pulverized by the blade 430 included in the mixer 420. The blade 430 may rotate by the driving force of the mixer 420, thereby pulverizing the solid seeds.

The solid seed supply unit 310 may further include a compressed air input line 440. The compressed air input line 440 may float solid seeds by spraying compressed air into the solid seed storage unit 410 where the solid seeds are stored. The compressed air is input to the solid seed storage unit 410 through the compressed air input line 440 to float the solid seeds, so that the solid seeds may be suppressed from being precipitated or hardened within the solid seed storage unit 410.

Hereinabove, although the present invention has been described with reference to exemplary embodiments and the accompanying drawings, the present invention is not limited thereto, but may be variously implemented by those skilled in the art to which the present invention pertains within the spirit of the present invention and the scope equivalent to the claims to be described below.

## Claims

1. A culturing apparatus, comprising:
a culturing chamber having a culturing space which is an internal space in which a culturing plate is accommodated;
a culturing door coupled to the culturing chamber to open and close an opening formed in the culturing chamber to allow the culturing plate to enter and exit;
a supply line unit formed in at least one of the culturing chamber and the culturing door to control a temperature, moisture supply, and seed supply of the culturing space, respectively; and
a fan part formed in at least one of the culturing chamber and the culturing door to spread a seed to the culturing plate.

2. The culturing apparatus of claim 1, wherein the culturing chamber includes a chamber circulation channel for circulating water outside the culturing space surrounding the culturing chamber.

3. The culturing apparatus of claim 1, wherein the culturing door includes a door circulation channel for circulating water outside the culturing space covered by the culturing door.

4. The culturing apparatus of claim 2, wherein the culturing door includes a door circulation channel for circulating water outside the culturing space covered by the culturing door, and
the culturing apparatus further includes a connection channel connecting the chamber circulation channel and the door circulation channel.

5. The culturing apparatus of claim 4, wherein the supply line unit includes a water supply line connected to at least one of the chamber circulation channel and the door circulation channel to supply water.

6. The culturing apparatus of claim 1, wherein the supply line unit includes a moisture supply line configured to supply the moisture to the culturing space.

7. The culturing apparatus of claim 6, wherein the water supply line is provided in plurality to have different heights.

8. The culturing apparatus of claim 1, wherein the supply line unit includes a seed supply line configured to supply the seed.

9. The culturing apparatus of claim 8, wherein the seed supply line is provided in plurality to have different heights.

10. The culturing apparatus of claim 1, wherein the fan part includes:
a first fan forming a flow in the culturing space; and
a second fan configured to scatter and supply the seed to the culturing plate.

11. The culturing apparatus of claim 10, wherein the fan part is provided so that a rotation speed is controlled.

12. A culturing system, comprising:
a culturing chamber having a culturing space which is an internal space in which a culturing plate is accommodated;
a culturing door coupled to the culturing chamber to open and close an opening formed in the culturing chamber to allow the culturing plate to enter and exit;
a seed supply unit configured to supply a seed to the culturing plate;
a fluid supply unit configured to supply water, moisture, and air to control a temperature and humidity of the culturing space; and
a processor electrically connected to the fluid supply unit,
wherein the processor controls the fluid supply unit to control at least one of the temperature and humidity of the culturing space.

13. The culturing system of claim 12, wherein the culturing system includes a pressure pump configured to control an internal pressure of the culturing space.

14. The culturing system of claim 13, wherein the processor is electrically connected to the pressure pump, and
the processor controls the pressure pump to lower the internal pressure of the culturing space to form a negative pressure and supply the seed to the culturing plate from the seed supply unit.

15. The culturing system of claim 13, wherein the seed supply unit includes a solid seed tank provided to store solid seed included in the seed.

16. The culturing system of claim 15, wherein the seed supply unit further includes a mixer configured to pulverize the solid seed.
